# EUROPEAN PATENT APPLICATION

(11) **EP 4 186 924 A1**
(43) Date of publication of application: **31.05.2023**
(21) Application number: 21846832.0
(22) Date of filing: 26.06.2021
(51) Int. Cl.: C07K 16/10, C12N 15/13, A61K 39/395, A61P 31/14

(54) **ANTI-NIPAH VIRUS MONOCLONAL ANTIBODY HAVING NEUTRALIZATION ACTIVITY AND APPLICATION**

(30) Priority: 22.07.2020 CN 202010711672
(71) Applicant: Academy of Military Medical Sciences, PLA, Beijing 100850 (CN)
(72) Inventor: CHEN, Wei, Beijing 100071 (CN); YU, Changming, Beijing 100071 (CN); LIU, Yujiao, Beijing 100071 (CN); FAN, Pengfei, Beijing 100071 (CN); ZHANG, Guanying, Beijing 100071 (CN); LI, Yaohui, Beijing 100071 (CN); LI, Jianmin, Beijing 100071 (CN); CHI, Xiangyang, Beijing 100071 (CN); HAO, Meng, Beijing 100071 (CN); FANG, Ting, Beijing 100071 (CN); DONG, Yunzhu, Beijing 100071 (CN); SONG, Xiaohong, Beijing 100071 (CN); CHEN, Yi, Beijing 100071 (CN); LIU, Shuling, Beijing 100071 (CN)
(74) Representative: dompatent von Kreisler Selting Werner - Partnerschaft von Patent- und Rechtsanwälten mbB
(86) International application number: PCT/CN2021/102585
(87) International publication number: WO 2022/017123

(57) **Abstract**

Disclosed in the present invention is an anti-Nipah virus monoclonal antibody having neutralization activity. The antibody consists of a monkey-derived variable region and a human constant region, and both light and heavy chains of the monkey-derived variable region have unique CDR regions. The antibody provided by the present invention has an excellent antigen binding capability, and has good binding activity with Bangladesh Nipah virus and Malaysia Nipah virus glycoprotein G. The antibody can effectively neutralize the Nipahpseudovirus. Moreover, the neutralization activity of the antibody is enhanced as the concentration of the antibody increases, and nearly 100% neutralization of the Nipahpseudovirus can be achieved at a concentration of 1 µg/mL. Also disclosed in the present invention is an application of the monoclonal antibody against the Nipah virus glycoprotein G in preparation of a Nipah virus treatment drug.

## Description

### TECHNICAL FIELD

The invention discloses a group of monoclonal antibodies, which belongs to the field of immunology and microbiology.

### BACKGROUND TECHNOLOGY

Nipah virus (NiV) is a single negative-stranded RNA virus, belonging to the genus Henipahvirus of the family Paramyxoviridae. NiVis a zoonotic virus that can be infected by direct contact and can cause fatal respiratory and neurological disease. The natural hosts of NiVis fruit bats.NiV can be transmitted through fruit bat-pig-human transmission and can also be directly transmitted to human from bats or from human itself.

NiV was first discovered in Malaysia. From September 1998 to April 1999, a number of pig farm staffs died from severe encephalitis and a large number of pigs died of illness in Perak, Malaysia. It was initially thought to be a Japanese encephalitis virus infection, but it was later found that this outbreak was significantly different from Japanese encephalitis in terms of susceptible population, infection rate, and infection mode. In addition, many of the patients had been vaccinated against Japanese encephalitis, so the researchers identified this as a new infectious disease. In this outbreak, both people and livestock showed acute respiratory syndrome, resulting in 256 infections, 105 deaths, and 1.16 million pig deaths. The epidemic further spread to a slaughter house in Singapore, causing 2,511 workers infected, and 1 died. In October 1999, researchers isolated the virus from the cerebrospinal fluid of a patient. Shortly afterward, NiV was isolated from the urine of Malaysian fruit bat, the natural host of NiV was identified. Subsequently, Nipah virus disease has been reported in India, Cambodia, Thailand and other countries. In recent years, Nipah virus disease has occurred many times in Bangladesh and India, causing hundreds of deaths, with a mortality rate of 50% to 100%. Studies on NiV have mainly focused on the Malaysia strain (NiV-MY) and the Bangladesh strain (NiV-BD).

### TECHNICAL PROBLEM

During the process of invading host cells, Nipah virus binds to the receptor ephrin-B2B3 through the viral surface glycoprotein G, which will activate the conformation change of fusion glycoprotein (F) , thereby mediate the fusion of the viral membrane and the cell membrane, and finally enable the viral genome to entry the cell. Both protein G and protein F are important targets in vaccine and antiviral drug development. There is currently no vaccine available for human use. As for drugs, only one monoclonal antibody, m102.4, has entered clinical trials. M102.4 is a human monoclonal antibody screened from recombinant human Fab phage display library. In addition to NiV, M102.4 can also effectively neutralize Hendravirus, another species belonging to the Henipavirus genus of the Paramyxoviridae family. In challenge protection experiments of ferrets and African green monkeys, m102.4 can achieve effective protection after Nipah virus challenge. In 2010, m 102.4 was administered as an emergency protective drug in two individuals at high risk of exposure, neither of whom developed symptoms of infection.

In view of the technical demand for therapeutic antibodies against Nipah virus in the art, the purpose of the invention is to provide candidate monoclonal antibodies targeting unique epitopes on protein G, and then provide its application in the preparation of a medicine for the treatment of Nipah virus disease.

### TECHNICAL SOLUTIONS

Based on the above purpose, the invention firstly provides a specific neutralizing antibody against Nipah virus glycoprotein G. The said antibody is monoclonal antibody, and the amino acid sequence of CDR(complementarity determining region )1, CDR2 ,CDR3 of the heavy chain variable region of the said antibody and the amino acid sequence of CDR1, CDR2, CDR3 of the light chain variable region of the said antibody are respectively shown as the following sequence combinations:
26-33, 51-58, 97-110 of SEQ ID NO:1 and 26-34, 52-54,91-100 of SEQ ID NO:3, or
26-33,51-58,97-105 of SEQ ID NO:5 and 27-37, 55-57, 94-107 of SEQ ID NO:7, or
26-33, 51-58, 97-110 of SEQ ID NO:9 and 26-34, 52-58, 97-105 of SEQ ID NO:11, or
26-33, 51-58, 97-108 of SEQ ID NO:13 and 27-37, 55-57, 94-102 of SEQ ID NO: 15.

In a preferred embodiment, the amino acid sequence of the heavy chain variable region of the said antibody and the amino acid sequence of the light chain variable region of the said antibody are respectively shown as any combination of the following sequences:
SEQ ID NO:1 and SEQ ID NOG (in the invention, the antibody with these variable regions is named as "1A9"), or
SEQ ID NO:5 and SEQ ID NO:7 (in the invention, the antibody with these variable regions is named as "1D11"), or
SEQ ID NO:9 and SEQ ID NO:11 (in the invention, the antibody with these variable regions is named as "1F9"), or
SEQ ID NO:13 and SEQ ID NO:15 (in the invention, the antibody with these variable regions is named as "2B6").

In a more preferred embodiment, the amino acid sequence of the heavy chain constant region of the said antibody is shown as SEQ ID NO: 17, and the amino acid sequence of the light chain constant region of the said antibody is shown as SEQ ID NO: 19 or SEQ ID NO:21.

Secondly, the invention also provides an isolated nucleic acid encoding the heavy chain and/or light chain of the said monoclonal antibody. The sequence of the isolated nucleic acid encoding the heavy chain variable region and/or the sequence of the isolated nucleic acid encoding the light chain variable region are respectively shown as any combination of the following sequences:
SEQ ID NO:2 and SEQ ID NO:4 (in the invention, the antibody with these variable regions is named as "1A9"), or
SEQ ID NO:6 and SEQ ID NO:8 (in the invention, the antibody with these variable regions is named as "1D11"), or
SEQ ID NO: 10 and SEQ ID NO: 12 (in the invention, the antibody with these variable regions is named as "1F9"), or
SEQ ID NO: 14 and SEQ ID NO: 16 (in the invention, the antibody with these variable regions is named as "2B6").

In a preferred embodiment, the isolated nucleic acid encoding the heavy chain constant region is shown as SEQ ID NO: 18, and the isolated nucleic acid encoding the light chain constant region is shown as SEQ ID NO:20 or SEQ ID NO:22.

Thirdly, the invention also provides a functional element expressing the above-mentioned isolated nucleic acid encoding the heavy chain and/or light chain of the said monoclonal antibody.

In a preferred embodiment, the functional element is a linear expression cassette.

In another preferred embodiment, the functional element is a mammalian expression vector.

Fourthly, the invention also provides a host cell comprising the above functional elements.

In a preferred embodiment, the host cell is Expi293F cell.

In another preferred embodiment, the host cell is CHO-S cell. In the invention, CHO-S cell can be used to construct stable expression cell lines to realize industrial production.

Finally, the invention provides an application of the above-mentioned monoclonal antibodies in the preparation of the therapeutic drug for Nipah virus disease .

### TECHNICAL EFFECT

The monoclonal antibodies against Nipah virus glycoprotein G in the invention are composed of monkey-derived variable region and human -derived constant region, and the monkey-derived light and heavy chains variable region have unique CDR regions. Antibodies disclosed in the invention exhibit excellent capacity of binding with antigen, and can effectively bind with glycoprotein (G) of Malaysia and Bangladesh Nipah virus. The antibodies can potently neutralize the pseudotyped Nipah virus. The neutralizing capacity of the antibody increased with the increase of antibody concentration, and nearly 100% inhibition against Nipah pseudo virus could be achieved at a concentration of 1 µg/mL. The invention also discloses the application of the said monoclonal antibodies against Nipah virus glycoprotein G in the preparation of the therapeutic drug for Nipah virus disease.

### BRIEF DESCRIPTION OF THE FIGURES

Figure 1. Sorting of rhesus monkey memory B cells;
Figure 2. Capillary electrophoresis of nested PCR products;
Figure 3. Distribution of ELISA OD₄₅₀₋₆₃₀ₙₘ values for screening of binding antibodies;
Figure 4. The curves of antibody binding with antigen in ELISA detection;
Figure 5. The neutralization capacity of mAbs against NiV-BD pseudovirus;
Figure 6. The neutralization curves of mAbs against NiV-MY pseudovirus;
Figure 7. The curves of mAbs competitively inhibiting to the binding of NiV-BD/MY G protein to receptor ephrin-B2/B3.

### EXAMPLES

The invention is further described below with reference to specific embodiments, and the advantages and characteristics of the invention will become clearer with the description. However, these embodiments are only exemplary, and do not constitute any limitation on the protection scope defined by the claims of the invention.

### Example 1. Screening and Preparation of Antibodies

### 1. Collection of blood samples

Female rhesus monkeys were immunized with adenovirus vector Nipah virus candidate vaccine, recombinant NiV G protein or recombinant HeV G protein three times by intramuscular injection on day 0, 28, and 49, respectively. Finally, blood samples of the rhesus monkey were collected on day 77.

### 2. Labeling of NiV-BD G with FITC

The NiV-BD G was labeled with fluorescein isothiocyanate (FITC) to sort antigen-specific memory B cells. Method is described as below:
1) FITC (SIGMA, F4274) is dissolved in dimethyl sulfoxide at a final concentration of 20 mg/mL. Take 100 µL of NiV-BD G (3.3 mg/mL) and add carbonate buffer (pH =9.6) to 400 µL.
2) Add 8 µL FITC to the NiV-BD G solution and incubate for 3 h at 4°C in the dark.
3) Buffer-exchange the protein into PBS using a 30 kDa centrifugal concentrator tube until the filtrate is transparent and colorless. Wrap the labeled protein in tin foil paper and store it at 4°C until use.

### 3. Flow sorting of memory B cells

PBMCs are isolated from blood samples using a Ficoll density gradient centrifugation method, details are described as follows:
1) Take fresh EDTA anticoagulant whole blood and dilute the whole blood with the same volume of PBS.
2) Add the separation solution to the centrifuge tube, and slowly spread the diluted blood sample above the surface of the separation solution and keep the interface between the two liquid surfaces clear. Separation solution, anticoagulated blood, PBS (or normal saline) volume is 1:1:1.
3) After Balancing, the tube is centrifuged at 800×g, 3rd gear acceleration and deceleration, room temperature, for 30 min. After centrifugation, the bottom of the tube is red blood cells, the middle layer is the separation solution, the top layer is the plasma/tissue homogenate layer. The thin and dense white film between the plasma layer and the separation solution layer is mononuclear cells (including lymphocytes and monocytes). Carefully pipette the mononuclear cells into another centrifuge tube.
4) Dilute the cells with PBS and gently invert and mix well. The tube is centrifuged at 300×g, room temperature, for 10 min. Discard supernatant and repeat twice. Finally, the lymphocytes were resuspended in PBS for later use.
5) Count 5×10⁵ cells in a volume of 50 µL PBS, add the five fluorescent dyes recommended in the following table 1, and incubate for 1 h at 4°C in the dark.

**Table 1. Fluorescent dyes used for cell sorting**

| Marker | Fluorescence | Company/Cat.No. | Volume |
|---|---|---|---|
| Antigen | FITC | SIGMA, F4274 | 4 µg |
| IgG | PE | BD, 555787 | 15µL |
| CD19 | APC-AF 700 | Beckman, IM2470 | 5µL |
| CD3 | PerCP | BD, 552851 | 10 µL |
| CD27 | PC7 | Beckman, A54823 | 10 µL |

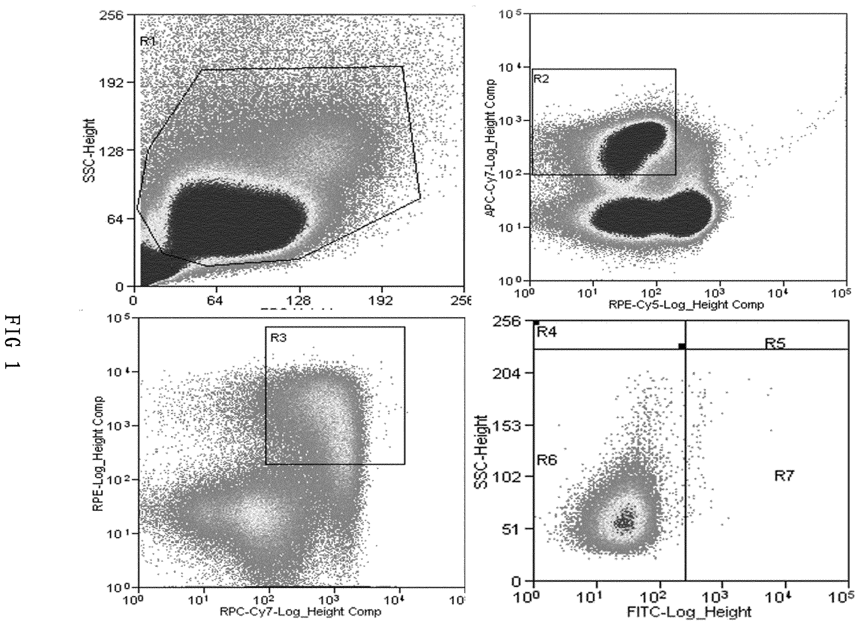

6) Wash cells 2-3 times with PBS containing 2% FBS and resuspend in 400 µL FPBS. Remove cell clusters with a 40 µm cell filter, and store at 4°C in the dark for sorting.
7) NiV-BD G-specific single memory B cells are sorted by a cell sorter (Beckman, MoFlo XDP) using a strategy of IgG⁺/CD3⁻/CD19⁺/CD27⁺/NiV-BD G⁺. Each single cell is directly sorted into 96-well plates contains 20U RNase inhibitor and 20µL RNase free water in each well. Store plates at -80°C. Cell sorting result is shown in Figure 1. Cells circled by R7 box in the figure are characterized by IgG⁺/CD3⁻/CDI9⁺/CD27⁺/NiV-BD G⁺, which are NiV-BD G-specific memory B cells.

### 4. Amplification of antibody genes by single cell PCR

### 1) Reverse transcription PCR

A total of 1124 NiV-BDG-specific memory B cells were obtained by flow sorting. The SuperScript III reverse transcription kit was used to perform reverse transcription polymerase chain reaction (PCR). The mixed system was prepared according to the instructions and directly added to 96-well plates containing single cells for PCR reaction. The reaction system and conditions are described as follows.

Reaction conditions: 42°C, 10 min; 25°C, 10 min; 50°C, 60 min; 94°C, 5 min. The reaction system is described as table 2.

**Table 2. Reaction system for reverse transcription PCR**

| Component | Volume |
|---|---|
| Template (sorted single cells) | 20 µL |
| Random primer | 3 µL |
| dNTP | 1µL |
| 10× buffer | 3 µL |
| 0.1MDTT | 1µL |
| MgCl₂ | 2µE |
| RNaseOUT | 1µL |
| SuperScript III | 0.5 µL |

### 2) Nested PCR

Reverse transcription products were used as the template, and two rounds of nested PCR reactions were performed to amplify H, κ, and λ genes. The detail process is described as follows.

The first-round nested PCR reaction system is listed in Table 3.

**Table 3. The reaction system for the first-round of nested PCR**

| Component | Volume |
|---|---|
| Template (Reverse transcription products) | 1µL |
| Mixed primers (H/κ/λ) | 1.5/1/1µL |
| dNTP | 2µL |
| 10× buffer | 2.5µL |
| TransStart Taq DNA polymerase | 0.5µL |
| RNase-free water | to 25µL |

The first-round of nested PCR reaction conditions: firstly pre-denaturation 5 min at 95°C; then 40 cycles of denaturation 30 s at 95 °C, annealing 30 s at 57°C and elongation 45 s at 72°C; finally elongation 10 min at 72°C.

The first-round nested PCR primers are listed in Table 4.

**Table 4. Primers for first-round nested PCR**

| Primer | Sequence |
|---|---|
| 5'VH1.L1 | ATGGACTKGACCTGGAGG |
| 5'VH2.L1 | ATGGACACGCTTTGCTCC |
| 5'VH3A.L1 | ATGGAGTTKGGGCTGAGCTG |
| 5'VH3B.L1 | ATGGAGTTTGKRCTGAGCTGG |
| 5'VH3C.L1 | ATGGAGTCRTGGCTGAGCTGG |
| 5'VH3D.L1 | ATGGAGTTTGTGCTGAGTTTGG |
| 5'VH4.L1 | ATGAAGCACCTGTGGTTC |
| 5'VHSA.L1 | ATGGGGTCAACTGCCATC |
| 5'VH5B.L1 | ATGGGGTCCACCGTCACC |
| 5'VH6.L1 | ATGTCTGTCTCCTTCCTCA |
| 5'VH7.L1 | ATGGACCTCACCTGGAGC |
| 3'IgG(Outer) | GGAAGGTGTGCACGCCGCTGGTC |
| 5'VK1A.L1 | ATGGACATGAGGGTCCCCGC |
| 5'VK1B.L1 | GGCTCCTKCTGCTCTGGCTC |
| 5'VK2L1 | ATGARGYTCCCTGCTCAG |
| 5'VK3.L1 | ATGGAARCCCCAGCWCAGC |
| 5'VK4.L1 | ATGGTGTCACAGACCCAAGTC |
| 5'VK5.L1 | ATGGCATCCCAGGTTCASC |
| 5'VK6A.L1 | ATGTTGTCTCCATCACAACTC |
| 5'VK6B.L1 | ATGGTGTCCCCATTGCAACTC |
| 5'VK7.L1 | ATGGGGTCCTGGGCTCC |
| 3'Kappa(Outer) | GTCCTGCTCTGTGACACTCTC |
| 5'VL1.L1 | ATGGCCTGGTYYCCTCTC |
| 5'VL2/7/10.L1 | ATGGCCTGGRCTCTGCTCC |
| 5'VL3A.L1 | ATGGCCTGGATTCCTCTC |
| 5'VL3B.L1 | ATGGCCTGGACCTTTCTC |
| 5'VL3C.L1 | ATGGCCTGGACCCCTCCC |
| 5'VL4A.L1 | ATGGCCTGGGTCTCCTTC |
| 5'VL4BL1 | ATGGCCTGGACCCCACTC |
| 5'VL5/11.L1 | ATGGCCTGGACTCCTCTC |
| 5'VL6.L1 | ATGGCCTGGGCTCCACTCC |
| 5'VL8.L1 | ATGGCCTGGATGATGCTTC |
| 5'VL9.L1 | ATGGCCTGGGCTCCTCTG |
| 3'Lamda(Outer) | TGTTGCTCTGTTTGGAGGG |

The second-round nested PCR reaction system is listed in Table 5.

**Table 5. The reaction system for the second-round of nested PCR**

| Component | Volume |
|---|---|
| Template (Products of the first-round nested PCR) | 1.6µL |
| Mixed primers (H/κ/λ) | 3.2/1.6/1.6µL |
| dNTP | 3.2 µL |
| 10× buffer | 4µL |
| TransStart Taq DNA polymerase | 0.8µL |
| RNase-free water | to 40µL |

The second-round nested PCR primers are listed in Table 6.

**Table 6. Primers for second-round nested PCR**

| | Primer | Sequence |
|---|---|---|
| H | 5'VH1A.SE | TGGCAGCAGCTACAGGTGC |
| | 5'VH1B.SE | TGACAGCAGCTACAGGCGC |
| | 5'VH1C.SE | TGGCAGCAGCAACAGGCAC |
| | 5'VH2. SE | GTCCCGTCCTGGGTCTTGTC |
| | 5'VH3A.SE | GCTGTTTGGAGAGGTGTCCAGTGTG |
| | 5'VH3B.SE | GCCATATTAGAAGGTGTCCAGTGTG |
| | 5'VH3C.SE | GCTCTTTTGAAAGGTGTCCAGTGTG |
| | 5'VH3D.SE | GCTATTTTAAGAGGTGTCCAGTGTG |
| | 5'VH3E.SE | GCTATTTTAAAAGGTGTCCAGTGTG |
| | 5'VH4.SE | AGCTCCCAGATGGGTCYTGTCC |
| | 5'VH5.SE | GCTGTTCTCCARGGAGTCTGTG |
| | 5'VH6.SE | GGCCTCCCATGGGGTGTC |
| | 5'VH7A.SE | GCAGCAACAGGTGCCCACTC |
| | 5'VH7B.SE | GCAGCAACAGGCACCCACTC |
| | 3'IgG(Inner) | GTTCAGGGAAGTAGTCCTTGAC |
| κ | 5'VK1/2.SE | CTCCCAGGTGCCAGATGTGA |
| | 5'VK1B.SE | GGTCCCTGGRTCCAGTGGG |
| | 5'VK3A.SE | TGGCTCCCAGGTACCACYGGA |
| | 5'VK3B.SE | TGGATCCCGGATGCCGCCG |
| | 5'VK3C.SE | TGGCTTCCGGATACCACTGGA |
| | 5'VK4.SE | CTGGATCTCTGGTGTCTGTGG |
| | 5'VKS.SE | CCTTTGGATCTCTGMTGCCAGG |
| | 5'VK6.SE | TGGGTTCCAGTCTCCAAGGG |
| | 5'VK7.SE | TGTGCTCCAGGCTGCAATGG |
| | 3'Kappa(Inner) | ATTCAGCAGGCACACAACAGAG |
| λ | 5'VL1A.SE | CTGTGCAGGGTCCTGGGCC |
| | 5'VL1B.SE | CTGCACAGGGTCCYGGGCC |
| | 5'VL2.SE | TCACTCAGGGCACAGGATCC |
| | 5'VL3A.SE | CGCCCTCTGCACAGTCTCTGTGG |
| | 5'VL3B.SE | CACTCTCTGCACAGGTTCCGTGG |
| | 5'VL4A.SE | TTCATTTTCTCCACAGGTCTCTGTG |
| | 5'VL4B.SE | CTTCACTGCAGAGGTGTCTCTC |
| | 5'VL5.SE | CACTGCACAGGTTCCCTCTC |
| | 5'VL6.SE | CTGCACAGGGTCTTGGGCTG |
| | 5'VL8.SE | GCTTATGGCTCAGGAGTGGA |
| | 3'Lamda(Inner) | AGACACACTAGTGTGGCCTTG |

The reaction conditions of the second-round of nested PCR are the same as those of the first-round of nested PCR.

### 3) Capillary electrophoresis

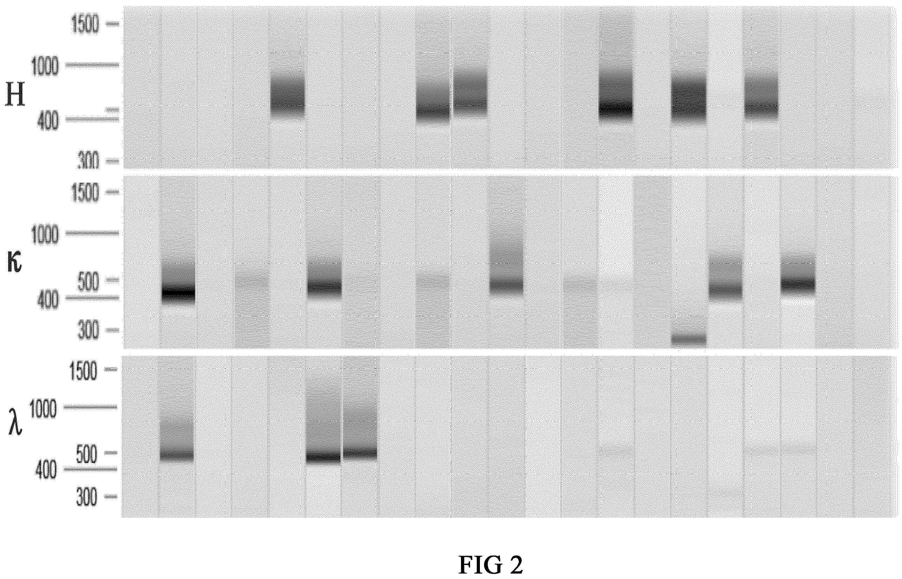

After the nested PCR, the amplified products were analyzed by capillary electrophoresis using the QIAxcel DNA Fast Analysis Cartridge. Positive clones with paired light and heavy chains were selected for sequencing, and the variable region sequences of the antibody were analyzed by Vector NTI software and IMGT website. The results of nested PCR capillary electrophoresis are shown in Figure 2.

### 5. Expression of antibodies using linear expression cassettes

Through the above single-cell PCR reaction, 254 paired antibody sequences were obtained, and the antibody was rapidly expressed by constructing linear expression cassettes.

Firstly, promoter-leader sequence fragments, constant region fragments (synthetized by Sangon Biotech, the heavy chain constant region sequence is shown as SEQ ID NO: 17, the DNA coding sequence is shown as SEQ ID NO: 18; the constant region sequence of the kappa light chain is shown as SEQ ID NO: 19, the DNA coding sequence is shown as SEQ ID NO:20; the constant region sequence of the lambda light chain is shown as SEQ ID NO:21, and the DNA coding sequence is shown as SEQ ID NO:22), and poly A-tail fragments (Genbank accession number: X03896.1) were obtained by PCR. Then amplify the antibody variable region fragments, and the PCR reaction system is listed in Table 7.

**Table 7. Reaction system for amplifying variable region fragments**

| Component | Volume |
|---|---|
| Template (Products of the second-round nested PCR) | 0.5µE |
| Mixed primers | 0.3µL |
| dNTP | 2µL |
| 10× buffer | 2µL |
| TransStart Taq DNA polymerase | 0.25µL |
| Deionized water | to 20 µL |

PCR reaction conditions: firstly pre-denaturation at 95°C for 5 min; then 30 cycles of 95°C, 30 s; 60°C, 30 s and 72°C, 30 s; finally elongation at 72°C for 10 min.

Take the amplified promoter-leader sequence fragment, constant region-poly A tail fragment and variable region fragment as templates, and use CMV-UP and TK-PolyA as primers, to perform overlapped extension PCR to amplify linear expression cassettes of H, κ and λ chains. PCR products were identified by nucleic acid electrophoresis. The reaction system for amplifying the full-length linear expression cassettes is listed in Table 8.

**Table 8. Reaction system for amplifying full-length linear expression cassettes**

| Component | Volume |
|---|---|
| Template 1 (promoter-leader sequence fragment) | 10ng |
| Template 2 (constant region-poly A tail fragment) | 10 ng |
| Template 3 (variable region fragment) | 0.5 µL |
| Upstream primer (CMV-UP) | 2.5 µL (10 µM) |
| Downstream primer (TK-Poly A) | 2.5 µL (10 µM) |
| dNTP | 4µL |
| 10× buffer | 5µL |
| TransStart Taq DNA polymerase | 1µL |
| Deionized water | to 50 µL |

PCR reaction conditions: pre-denaturation at 95°C for 5 min; 95°C, 30 cycles of 30 s; 60°C, 30 s; 72°C and 3 min; finally elongation at 72°C for 10 min.

PCR reaction products were directly recovered with the OMEGA kit and quantified with Nano (GE Healthcare). One day before transfection, 2×10⁴ cells in 150 µL medium were seeded into each well of 96-well plates. On the day of transfection, took 0.2 µg of each light and heavy chain, added 0.8 µL of Turbofect transfection reagent, diluted to 40 µL with DMEM medium, and incubated at room temperature for 15 min after mixing. The mixtures were slowly added dropwise to 96-well plates and then cultured in a 37°C incubator for 48 h.

6. Screening of antibodies with binding capacity by ELISA
1) One day before the experiment, microplates are coated with 100 µL of NiV-BD G at 1 µg/mL and incubated overnight at 4°C in a humid box.
2) On the following day, plates are washed 5 times with a plate washer (BIO-TEK, 405_LS). After adding 100 µL of blocking buffer to each well, plates are incubated at 37°C for 1 h.
3) After washed 5 times, plates are added 100 µL of the transfected cell culture supernatant and then incubated at 37°C for 1 h.
4) After washed, plates are added 100 µL of HRP-labelled goat anti-human IgG - (Abeam, Ab97225) at a dilution of 1:10,000, and then incubated at 37°C for 1 h.
5) After washed, plates are added 100 µL of TMB substrate for 6 min in the dark at room temperature, followed by addition of 50 µL stop solution. Optical density at a 450-630nm is read on a microplate reader.

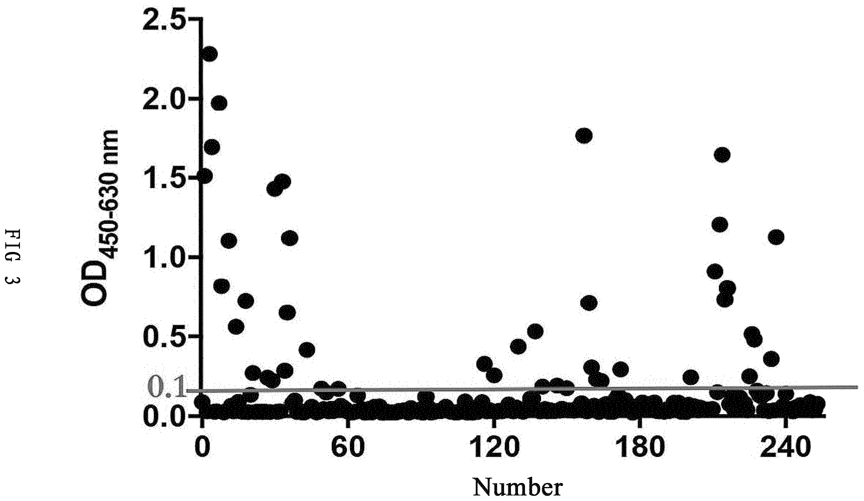

Results: Taking 0.1 as the cutoff value of optical density, Fifty-nine antibodies that can specifically bind to NiV-BDG were screened from 254 amplified positive clones. These antibodies were further expressed, purified and verified. Distribution of OD values for ELISA screening of antibodies with binding capacity is shown in Figure 3.

### 7. Construction of the expression plasmid and preparation of the antibody

The expression plasmids are constructed and then the antibodies are prepared by expression. The method is described as follows:
1) Full-length genes of heavy chain and light chain linear expression cassettes are digested with EcoR I (NEB, R3101) and *Not* I (NEB, R3189),then ligated into pcDNA3.4 expression plasmids.
2) 15 µg of pcDNA3.4-H and 15 µg of pcDNA3.4-L are co-transfected into 30 mL Expi293 system (Life, A14524), and cells are cultured at 125 rpm, 5% CO₂ for 72 h.
3) The expression supernatant is collected by centrifugation at 3000 ×g for 10 min. Antibodies are purified using a rProtein A column. Antibodies are buffer-exchanged into PBS and then quantified by BCA protein quantification kit (Thermo Scientific, 23225).

### Example 2. Detection of the binding capacity of the antibody by ELISA

1) One day before the assay, microplates are coated with 100 µL of NiV-BD G or NiV-MY G (NiV-BD G, Genbank: AY988601.1; NiV-MY G, Genbank: FN869553.1) at 1 µg/mL and incubated overnight at 4°C.
2) On the following day, after washing plates, adding 100 µL of blocking buffer is added into each well, plates are incubated at room temperature for 1 h.
3) Wash plates by plate-washer, then add 150 µL of purified antibodies at a concentration of 20 µg/mL to the first well, and add 100 µL of dilution solution to the remaining wells. Transfer 50 µL from the first well to the second well, and so on, dilute at a gradient of 1:3, with a final volume of 100 µL per well. Incubate plates for 1 h at room temperature.
4) Wash plates, add 100 µL of HRP -labelled goat anti-human IgG (Abeam, Ab97225) at a dilution of 1:10,000 into each well, and then incubated at room temperature for 1 h.
5) Wash plates, then add 100 µL of TMB substrate per well, and allow color reacting for 6 min in the dark at room temperature, followed by addition of 50 µL stop solution, finally read optical density at a 450-630nm on a microplate reader.

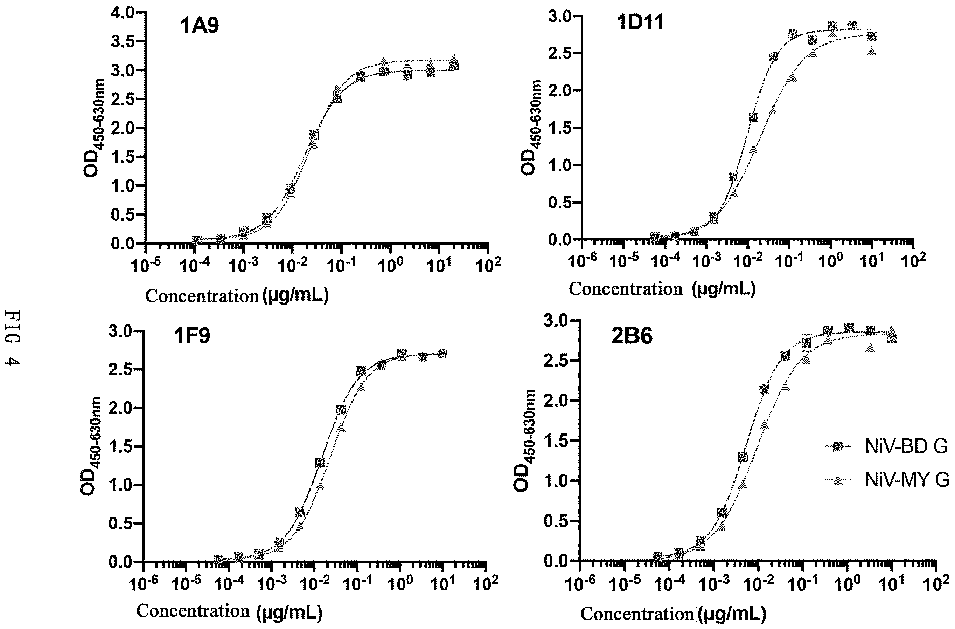

As shown in Figure 4, antibodies have good binding capacity to the G protein of NiV-BD and NiV-MY The half effective concentration (EC₅₀) values of antibody 1A9 are 18.26 ng/mL and 22.81 ng/mL, respectively; the EC₅₀ values of antibody 1D11 are 9.91 ng/mL and 20.2 ng/mL, respectively; the EC₅₀ values of antibody 1F9 are 15.04 ng/mL and 23.56 ng/mL, respectively; the EC₅₀ values of antibody 2B6 are 5.40 ng/mL and 9.56 ng/mL, respectively.

The above-mentioned four antibodies are sequenced. The nucleotide sequences of the heavy chain variable region of 1A9 is shown as SEQ ID NO:2; the nucleotide sequences of the light chain variable region of 1A9 is shown as SEQ ID NO:4; the amino acid sequences of the heavy chain variable regions of 1A9 is shown as SEQ ID NO: 1; the amino acid sequences of the light chain variable regions of 1A9 is shown as SEQ ID NO:3; further analysis on the amino acid sequences of the heavy chain and the light chain variable region shows, the amino acid sequences of the CDR1, CDR2 and CDR3 region of the heavy chain variable region are respectively shown as 26-33, 51-58, and 97-110 of SEQ ID NO:1, the amino acid sequences of CDR1, CDR2 and CDR3 regions of the light chain variable region are respectively shown as 26-34, 52-54, and 91-100 of SEQ ID NO:3.

The nucleotide sequences of the heavy chain variable region of 1D11 is shown as SEQ ID NO:6; the nucleotide sequences of the light chain variable region of 1D11 is shown as SEQ ID NO:8; the amino acid sequences of the heavy chain variable regions of 1D11 is shown as SEQ ID NO:5; the amino acid sequences of the light chain variable regions of 1D11 is shown as SEQ ID NO:7; further analysis on the amino acid sequences of the heavy chain and the light chain variable region shows, the amino acid sequences of the CDR1, CDR2 and CDR3 region of the heavy chain variable region are respectively shown as 26-33, 51-58, and 97-105 of SEQ ID NO:5, the amino acid sequences of CDR1, CDR2 and CDR3 regions of the light chain variable region are respectively shown as 27-37, 55-57, and 94-107 of SEQ ID NO:7.

The nucleotide sequences of the heavy chain variable region of 1F9 is shown as SEQ ID NO: 10; the nucleotide sequences of the light chain variable region of 1F9 is shown as SEQ ID NO: 12; the amino acid sequences of the heavy chain variable regions of 1F9 is shown as SEQ ID NO:9; the amino acid sequences of the light chain variable regions of 1F9 is shown as SEQ ID NO: 11; further analysis on the amino acid sequences of the heavy chain and the light chain variable region shows, the amino acid sequences of CDR1, CDR2 and CDR3 region of the heavy chain variable region are respectively shown as 26-33, 51-58, and 97-110 of SEQ ID NO:9, the amino acid sequences of CDR1, CDR2 and CDR3 regions of the light chain variable region are respectively shown as 26-34, 52-58, and 97-105 of SEQ ID NO: 11.

The nucleotide sequences of the heavy chain variable region of 2B6 is shown as SEQ ID NO: 14; the nucleotide sequences of the light chain variable region of 2B6 is shown as SEQ ID NO: 16; the amino acid sequences of the heavy chain variable regions of 2B6 is shown as SEQ ID NO: 13; the amino acid sequences of the light chain variable regions of 2B6 is shown as SEQ ID NO: 15; further analysis on the heavy chain and light chain variable region amino acid sequences shows, the amino acid sequences of CDR1, CDR2 and CDR3 region of the heavy chain variable region are respectively shown as 26-33, 51-58, and 97-108 of SEQ ID NO: 13, the amino acid sequences of CDR1, CDR2 and CDR3 regions of the light chain variable region are respectively shown as 27-37, 55-57, and 94-102 of SEQ ID NO: 15.

The above four monoclonal antibodies have the same human heavy chain and light chain constant regions. The sequence of the polynucleotide encoding the heavy chain constant region is shown as SEQ ID NO: 18, and the sequence of the polynucleotide encoding the light chain constant region is shown as SEQ ID NO:20 or SEQ ID NO:22, the amino acid sequence of the heavy chain constant region is shown as SEQ ID NO: 17, and the amino acid sequence of the light chain constant region is shown as SEQ ID NO: 19 or SEQ ID NO:21.

### Example 3. Pseudovirus neutralization assay to evaluate the neutralizing capacity of the antibodies

Package of human immunodeficiency virus (HIV)-backbone NiV-BD andNiV-MY pseudoviruses to evaluate the neutralizing capacity of monoclonal antibodies in vitro (Dimple Khetawat, C.C.B., A functional henipavirus envelope glycoprotein pseudotyped lentivirus assay system. Virology Journal 2010. 7(312)). Method is as below:
1) Dilute antibodies with DMEM medium, add 75 µL of antibody at 5µg/mL to the first well of 96-well culture plates, and add 50 µL of DMEM medium to the remaining wells.
2) Transfer 25 µL of liquid from the first well into the second well, mix well, and so on, dilute at a ratio of 1:3, and the final volume of each well is 50 µL. Add 50 µL pseudovirus to each well and incubate at 37°C for 1 h.
3) Count 293T cells and seed 100 µL cells at a density of 2×10⁵ cells/mL to each well. Place the culture plate in a 37°C incubator for 36-48 h.
4) Take out plates, then carefully remove the medium. Add 100 µL of cell lysate to each well and shake at 400 rpm for 15 min on a shaker. Centrifuge at 3000 rpm for 10 min at room temperature. After mixing the lyophilized detection substrate and buffer of the luciferase detection system (Promega, E1501), then fill them in the GLOMAX 96 Microplate Luminometer (Promega) detection loops. Transfer 20 µL of the lysis supernatant and read the fluorescence value. Calculate the protection rate of the antibody on the cells.

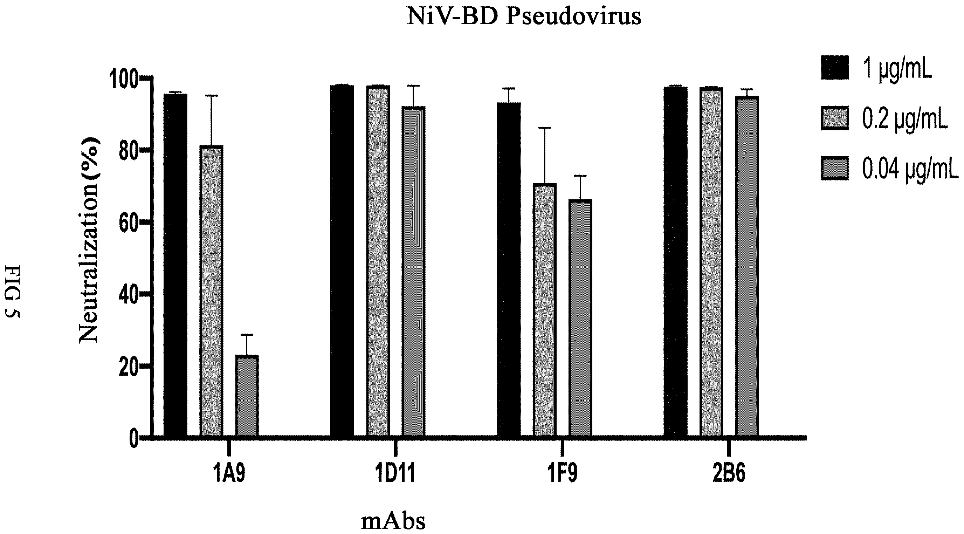

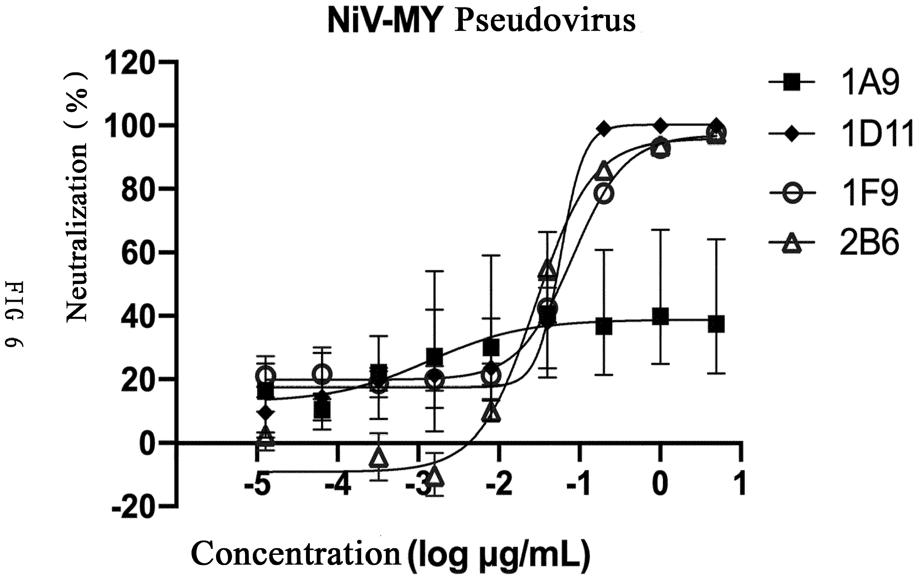

The results are shown in Figure 5 and Figure 6. The antibodies can effectively neutralize HIV-NiV-BD/MY pseudoviruses in vitro. The neutralizing capacity of the antibody increased with the increase of its concentration. As for 1D11, 1F9 and 2B6, nearly 100% neutralization against the two pseudotyped Nipah viruses could be achieved at a concentration of 1 µg/mL. However. 1A9 can only neutralize the NiV-BD pseudovirus, and has poor neutralization effect against the NiV-MY pseudovirus. For NiV-BD pseudovirus, monoclonal antibodies 1D11 and 2B6 have higher neutralization capacity than 1A9 and 1F9, and their neutralization rate can still reach over 95% at a concentration of 0.04 µg/mL; for NiV-MY pseudovirus, the half inhibiting concentration (IC₅₀) values of 1D11, 1F9 and 2B6 are 57.87 ng/mL, 81.41 ng/mL and 28.26 ng/mL, respectively.

### Example 4. Competition experiment

The capacity of monoclonal antibody inhibiting the binding of Nipah virus G protein with the receptor was evaluated through Luminex microsphere competition inhibition assay. The method is described as follows:
1) Add 10 µL of 10 µg monoclonal antibody to the first well, and then dilute it by two times successively.
2) Add 1.25 ng of receptor ephrin-B2 or ephrin-B3 to each well in a volume of 10 µL. Add 10 µL of prepared microspheres (containing 1500 NiV-BD/MY G-coupled microspheres respectively) to each well, and incubate on a shaker for 60 min.
3) Add 10 µL of SAPE (concentration of 12 µg/mL) to each well and incubate on a shaker for 30 min.
4) Wash 3 times with 100 µL assay buffer and read by Luminex MAGPIX instrument.

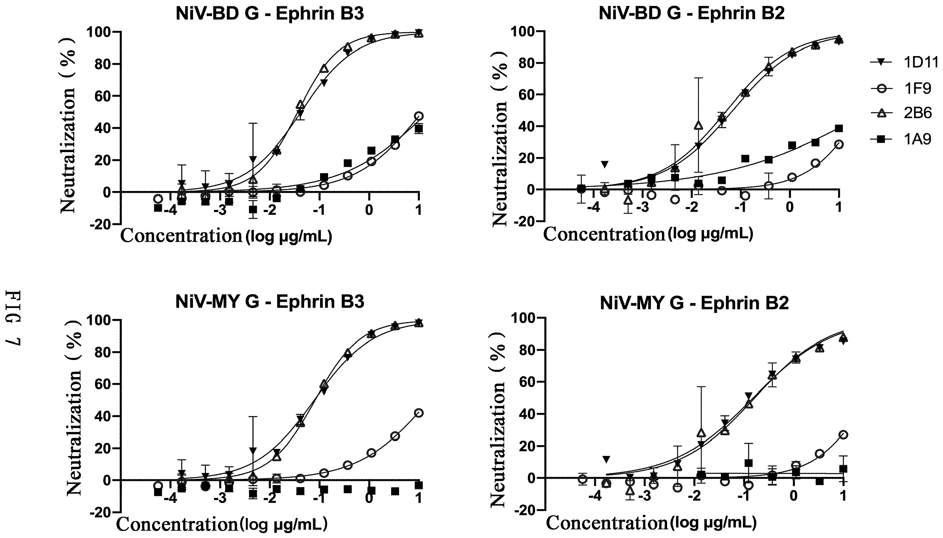

The curves of antibodies competitively inhibiting the binding of Nipah virus G protein with receptor ephrin-B2/B3 are shown in Figures 7.The results show that 1D11 and 2B6 can effectively inhibit the binding of Nipah virus G protein with receptor ephrin-B2/B3 binding. The IC₅₀ values of 1D11 and 2B6 for inhibiting the binding of Nipah virus G protein with the receptor ephrin-B2B3 ranged from 0.04 µg/mL to 0.16 µg/mL, suggesting that antibodies 1D11 and 2B6 are likely to play a neutralizing role by inhibiting the binding of Nipah virus G protein with receptor ephrin-B2B3, while 1A9 and 1F9 may have other neutralizing mechanisms.

### INDUSTRIAL APPLICABILITY

The invention provides a series of anti-Nipah virus monoclonal antibodies with neutralizing activity and their application in the preparation of medicines. The monoclonal antibodies are easy to be industrially produced and have industrial practicability.

## Claims

1. A monoclonal antibody against Nipah virus glycoprotein G, wherein the amino acid sequence of CDR1, CDR2 ,CDR3 of the heavy chain variable region of the said antibody and the amino acid sequence of CDR1, CDR2 , CDR3 of the light chain variable region of the said antibody are shown respectively as the following sequence combinations:
26-33, 51-58, 97-110 of SEQ ID NO:1 and 26-34, 52-54,91-100 of SEQ ID NO:3, or
26-33, 51-58,97-105 of SEQ ID NO:5 and 27-37, 55-57, 94-107 of SEQ ID NO:7, or
26-33, 51-58, 97-110 of SEQ ID NO:9 and 26-34, 52-58, 97-105 of SEQ ID NO:11, or
26-33, 51-58, 97-108 of SEQ ID NO: 13 and 27-37, 55-57, 94-102 of SEQ ID NO: 15.

2. The monoclonal antibody of claim 1, wherein the amino acid sequence of the heavy chain variable region of the said antibody and the amino acid sequence of the light chain variable region of the said antibody are respectively shown as the following sequence combinations:
SEQ ID NO:1 and SEQ ID NO:3, or
SEQ ID NO:5 and SEQ ID NO:7, or
SEQ ID NO:9 and SEQ ID NO:11, or
SEQ ID NO:13 and SEQ ID NO: 15.

3. The monoclonal antibody of claim 2, wherein the amino acid sequence of the heavy chain constant region of the said antibody is shown as SEQ ID NO: 17, and the amino acid sequence of the light chain constant region of the said antibody is shown as SEQ ID NO:19 or SEQ ID NO:21.

4. An isolated nucleic acid encoding the heavy chain and/or the light chain of the monoclonal antibody of any one of the claims 1-3, wherein the sequence of the isolated nucleic acid encoding the heavy chain variable region and/or the sequence of the isolated nucleic acid encoding the light chain variable region are respectively shown as the following sequence combinations:
SEQ ID NO:2 and SEQ ID NO:4, or
SEQ ID NO:6 and SEQ ID NO:8, or
SEQ ID NO: 10 and SEQ ID NO: 12, or
SEQ ID NO: 14 and SEQ ID NO: 16.

5. The isolated nucleic acidof claim 4, wherein the sequence of the isolated nucleic acid encoding the heavy chain constant region is shown as SEQ ID NO: 18, and the sequence of the isolated nucleic acid encoding the light chain constant region is shown as SEQ ID NO:20 or SEQ ID NO:22.

6. A functional element expressing the isolated nucleic acid of claim 5.

7. The functional element of claim 6, wherein the functional element is a linear expression cassette or a mammalian expression vector.

8. A host cell comprising the functional element of claim 7.

9. The host cell of claim 8, wherein the cell is Expi 293F cell or CHO-S cell.

10. Application of the monoclonal antibody of any of claims 1-3 in the preparation of a therapeutic drug for Nipah virus disease.


### SEQUENCE LISTING

<110>  ACADEMY OF MILITARY MEDICAL SCIENCE, PLA

<120>  Anti-Nipah Virus Monoclonal Antibody Having Neutralization Activity and
Application

<160>  22

<170>  SIPOSequenceListing 1.0

<210>  1
<211>  121
<212>  PRT
<213>  Macaca mulatta

<400>  1
Val Gln Leu Gln Glu Ser Gly Pro Gly Leu Val Lys Pro Ser Glu Thr
1               5                   10                  15
Leu Ser Leu Thr Cys Ala Val Ser Gly Asp Ser Ile Ser Thr Gly Tyr
            20                  25                  30
Asp Trp Thr Trp Ile Arg Gln Pro Pro Gly Lys Gly Leu Glu Trp Ile
        35                  40                  45
Gly Tyr Ile Gly Gly Ser Ser Gly Asn Thr Asn Tyr Asn Pro Ser Leu
    50                  55                  60
Lys Ser Arg Val Thr Ile Ser Lys Asp Thr Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Asp Gly Thr Arg Val Tyr Tyr Tyr Gly Leu Asp Ser Trp Gly

            100                 105                 110
Gln Gly Val Val Val Thr Val Ser Ser
        115                 120

<210> 2
<211> 364
<212> DNA
<213> Macaca mulatta

<400> 2
gtgcagctgc aggagtcggg cccagggctg gtgaagcctt cggagaccct gtccctcacc 60
tgcgctgtct ctggtgactc catcagcact ggttatgact ggacctggat ccgccagccc 120
ccagggaaag ggctggagtg gattgggtat atcggtggta gtagtggtaa caccaactac 180
aatccctccc tcaagagtcg agtcaccatt tcaaaagaca cgtccaagaa ccagttctcc 240
ctgaagctga gttctgtgac cgccgcggac accgccgtgt attactgtgc gagagatggc 300
acccgggtgt attactacgg tttggattcc tggggccaag gggtcgtcgt caccgtctcc 360
tcag 364

<210> 3
<211> 110
<212> PRT
<213> Macaca mulatta

<400> 3
Glu Thr Val Val Thr Gln Glu Pro Ser Leu Ser Val Ser Pro Gly Gly
1               5                   10                  15
Thr Val Thr Leu Thr Cys Gly Leu Ser Ser Gly Ser Val Ser Thr Ser
            20                  25                  30
Asn Tyr Pro Ser Trp Phe Gln Gln Thr Pro Gly Gln Ala Pro Arg Met
        35                  40                  45
Leu Ile Tyr Ser Thr Asn Ile Arg Pro Ser Gly Val Pro Asn Arg Phe
    50                  55                  60
Ser Gly Ser Ile Leu Gly Asn Lys Ala Ala Leu Thr Ile Thr Gly Ala
65                  70                  75                  80
Gln Ala Asp Asp Glu Ser Asp Tyr Tyr Cys Phe Phe Ser Met Val Ser
                85                  90                  95
Gly Thr Ser Val Phe Gly Gly Gly Thr Arg Leu Thr Val Leu
            100                 105                 110

<210> 4
<211> 331
<212> DNA
<213> Macaca mulatta

<400> 4
gagactgtgg tgacccagga gccatcgttg tcagtgtccc ctggaggaac agtcacactc 60
acctgtggcc tgagctctgg ctcagtctct accagcaact accccagctg gttccagcag 120
accccaggcc aggctccacg catgctcatc tacagtacaa atattcgccc ttctggggtc 180
cctaatcgct tctctggctc catccttggg aacaaagctg ccctcaccat cacgggggct 240
caggcagacg atgaatctga ttactactgt ttcttctcca tggttagtgg cacttcggtt 300
ttcggcggag ggacccggct gaccgtccta g 331

<210> 5
<211> 116
<212> PRT
<213> Macaca mulatta

<400> 5
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Asp Met Ser Trp Val Arg Gln Ala Pro Gly Lys Gly Leu Glu Trp Val
        35                  40                  45
Ser Ser Ile Ser Ser Val Thr Ser Tyr Ile Ser Tyr Ala Asp Ser Val
    50                  55                  60
Lys Gly Arg Phe Thr Ile Ser Arg Asp Asn Ala Lys Asn Ser Leu Ser
65                  70                  75                  80
Leu Gln Met Asn Ser Leu Lys Thr Glu Asp Thr Ala Val Tyr Tyr Cys
                85                  90                  95
Thr Thr Glu Asn Trp Asn Phe Asp Phe Trp Gly Gln Gly Val Leu Val
            100                 105                 110
Thr Val Ser Ser
        115

<210> 6
<211> 349
<212> DNA
<213> Macaca mulatta

<400> 6
gaggtgcaac tggtggagtc tgggggaggc ttggtccagc ctggagggtc cctgagactc 60
tcctgtgcag cctctggatt caccttcagt agttacgaca tgagctgggt ccgccaggct 120
ccgggaaagg ggctggagtg ggtctcatcc attagtagtg ttactagtta catatcctac 180
gctgactccg tgaagggccg attcaccatc tccagagaca acgccaagaa ctcgctgtct 240
ctgcaaatga acagcctgaa aaccgaggac acggccgtat attactgtac tactgaaaac 300
tggaactttg acttctgggg ccagggagtc ctggtcaccg tctcctcag 349

<210>  7
<211>  113
<212>  PRT
<213> Macaca mulatta

<400>  7
Asp Val Val Met Thr Gln Ser Pro Leu Ser Leu Ala Thr Thr Pro Gly
1               5                   10                  15
Gln Pro Ala Ser Ile Ser Cys Arg Ser Ser Gln Ser Leu Val His Ser
            20                  25                  30
Asn Gly Asn Thr Tyr Leu Ser Trp Tyr Gln Gln Lys Pro Gly Gln Pro
        35                  40                  45
Pro Arg Leu Leu Ile Tyr Glu Val Ser Asn Arg Asp Ser Gly Val Pro
    50                  55                  60
Asp Arg Phe Ser Gly Ser Gly Ala Gly Thr Asp Phe Thr Leu Lys Ile
65                  70                  75                  80
Ser Arg Val Glu Ala Glu Asp Val Gly Val Tyr Tyr Cys Gly Gln Gly
                85                  90                  95
Thr His Trp Pro Pro Phe Thr Phe Gly Pro Gly Thr Lys Leu Asp Ile
            100                 105                 110
Lys

<210>  8
<211>  340
<212>  DNA
<213>  Macaca mulatta

<400>  8
gatgttgtga tgactcagtc tccactctcc ctggccacca cccctggaca gccggcctcc      60
atctcctgca ggtctagtca aagccttgta cacagtaatg gaaacaccta cttgagttgg     120
tatcagcaga agccaggcca acctccaagg ctcctgattt atgaggtttc taaccgggac     180
tctggggtcc cagacagatt cagcggcagt ggggcaggga cagatttcac actgaaaatc     240
agcagagtgg aggctgagga tgttggggtt tattactgcg ggcaaggtac acactggcct     300
ccgttcactt tcggccccgg gaccaaactg gatatcaaac                           340

<210>  9
<211>  121
<212>  PRT
<213>  Macaca mulatta

<400>  9
Gln Val Gln Leu Gln Glu Ser Gly Pro Gly Leu Val Lys Pro Ser Glu
1               5                   10                  15

Thr Leu Ser Leu Ser Cys Ala Val Ser Gly Gly Ser Phe Ser Asp Tyr
            20                  25                  30
Trp Trp Gly Trp Ile Arg Gln Ser Pro Gly Lys Gly Leu Glu Trp Ile
        35                  40                  45
Gly Ser Ile Tyr Gly Ser Ser Gly Arg Thr Ala Tyr Ser Pro Ser Leu
    50                  55                  60
Lys Ser Arg Ala Thr Ile Ser Arg Asp Thr Ser Lys Asn Gln Leu Phe
65                  70                  75                  80
Leu Arg Leu Thr Ser Val Thr Ala Ala Asp Thr Gly Val Tyr Tyr Cys
                85                  90                  95
Thr Asn Glu Arg Phe Tyr Gly Asp Ser Asn Arg Phe Asp Val Trp Gly
            100                 105                 110
Pro Gly Val Leu Val Thr Val Ser Ser
        115                 120

<210>  10
<211>  364
<212>  DNA
<213>  Macaca mulatta

<400>  10
caggtgcagc tgcaggagtc gggcccagga ctggtgaagc cttcggagac cctgtccctc      60
tcctgcgctg tctctggtgg ctccttcagc gattactggt ggggctggat ccgccagtcc     120
ccagggaagg ggctggagtg gattgggagt atctatggta gtagtgggcg aaccgcatac     180
agcccctccc tcaaaagtcg agccaccatt tcaagagaca cgtccaagaa ccagctcttc     240
ctgaggctga cctctgtgac cgccgcggac acgggagtct actactgtac caacgagagg     300
ttctacggtg acagcaaccg gttcgatgtc tggggcccgg gagtcctggt caccgtctcc     360
tcag                                                                  364

<210>  11
<211>  115
<212>  PRT
<213>  Macaca mulatta

<400>  11
Gln Pro Val Leu Thr Gln Pro Thr Ser Leu Ser Ala Ser Pro Gly Ala
1               5                   10                  15
Ser Ala Arg Leu Thr Cys Thr Leu Ser Ser Gly Ile Ser Val Gly Asn
            20                  25                  30
Tyr Arg Ile Phe Trp Tyr Gln Gln Lys Pro Gly Ser Pro Pro Arg Tyr
        35                  40                  45
Leu Leu Asn Tyr His Thr Asp Ser Asp Lys His Gln Gly Ser Gly Val
    50                  55                  60
Pro Ser Arg Phe Ser Gly Ser Lys Asp Ala Ser Ala Asn Ala Gly Ile

65                  70                  75                  80
Leu Leu Ile Ser Gly Leu Gln Ser Glu Asp Glu Ala Asp Tyr Tyr Cys
                85                  90                  95
Met Ile Trp His Asn Asn Val Asp Val Phe Gly Gly Gly Thr Lys Leu
            100                 105                 110
Thr Val Leu
        115

<210>  12
<211>  346
<212>  DNA
<213>  Macaca mulatta

<400>  12
cagcctgtgc tgactcagcc aacctccctc tcagcatctc ctggagcatc agccagactc      60
acctgcacct tgagcagtgg catcagtgtt ggtaattaca ggattttctg gtaccagcag     120
aagccaggga gtcctccccg gtatcttctg aactaccaca cagactcaga taagcaccag     180
ggctctggag tccccagtcg cttctctgga tccaaagatg cttcggccaa tgcagggatt     240
ttactcatct ctgggctcca gtctgaggat gaggctgact attactgtat gatttggcac     300
aacaatgttg atgtgttcgg aggtggcacc aagttgaccg tcctcg                    346

<210>  13
<211>  119
<212>  PRT
<213>  Macaca mulatta

<400>  13
Gln Val Gln Leu Val Gln Ser Gly Ala Glu Val Lys Lys Pro Gly Ala
1               5                   10                  15
Ser Val Lys Leu Ser Cys Lys Ala Ser Gly Tyr Thr Phe Thr Ser Phe
            20                  25                  30
Tyr Ile Asn Trp Val Arg Gln Ala Pro Gly Gln Val Leu Glu Trp Met
        35                  40                  45
Gly Trp Ile Asn Pro Ser Asn Gly Asp Thr Gly Tyr Ala Gln Lys Phe
    50                  55                  60
Gln Gly Arg Val Thr Met Ser Arg Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Glu Thr Val Gly Ser Gly Thr Phe Asp Cys Trp Gly Gln Gly
            100                 105                 110
Val Leu Val Thr Val Ser Ser
        115

<210> 14
<211> 358
<212> DNA
<213> Macaca mulatta

<400> 14
caggtccagc tggtgcagtc cggggctgag gtgaagaagc ctggggcctc agtgaaactc 60
tcctgcaagg cttctggtta cacctttacc agcttctata taaactgggt gagacaggcc 120
cctggacaag tgcttgagtg gatgggatgg attaacccta gcaatggtga tacaggctac 180
gcacagaaat tccagggcag agtcaccatg tccagggaca cgtcgacgag tacagcctac 240
atggagctga gcagcctgag atctgaggac acggccgtgt attattgtac gagagagact 300
gttggtagtg gaacctttga ctgctggggc cagggagtcc tggtcaccgt ctcctcag 358

<210> 15
<211> 112
<212> PRT
<213> Macaca mulatta

<400> 15
Asp Ile Val Met Thr Gln Thr Pro Leu Ser Leu Ser Val Thr Leu Gly
1               5                   10                  15
Glu Pro Ala Ser Ile Ser Cys Arg Ser Ser Gln Ser Leu Val Tyr Ser
            20                  25                  30
Ala Gly Lys Thr Tyr Leu Asp Trp Tyr Leu Gln Lys Pro Gly Gln Ser
        35                  40                  45
Pro Gln Leu Leu Ile Tyr Leu Val Ser Lys Arg Ala Ser Gly Val Pro
    50                  55                  60
Asp Lys Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Arg Ser Pro Leu Thr Phe Gly Gly Gly Thr Lys Val Glu Leu Lys
            100                 105                 110

<210> 16
<211> 337
<212> DNA
<213> Macaca mulatta

<400> 16
gatattgtga tgacccagac tccactctcc ctgtccgtca cccttggaga gccggcctcc 60
atctcctgca ggtctagtca gagccttgta tacagtgctg gaaaaaccta tttggattgg 120
tacctgcaga agccaggcca gtctccacag ctcctaatat atttggtttc caaacgggcc 180
tctggagtcc cagacaagtt cagtggcagt gggtcaggca ctgatttcac actgaaaatc 240

agcagggtgg aggctgagga tgttggggtt tattactgca tgcaagctct acggtctccg 300
ctcactttcg gcggagggac caaggtggag ctcaaac 337

<210> 17
<211> 330
<212> PRT
<213> Homo sapiens

<400> 17

<210>  18
<211>  990
<212>  DNA
<213>  Homo sapiens

<400>  18
gcctccacca agggcccatc ggtcttcccc ctggcaccct cctccaagag cacctctggg      60
ggcacagcag ccctgggctg cctggtcaag gactacttcc ccgaaccggt gacggtgtcg     120
tggaactcag gcgccctgac cagcggcgtg cacaccttcc cggctgtcct acagtcctca     180
ggactctact ccctcagcag cgtggtgacc gtgccctcca gcagcttggg cacccagacc     240
tacatctgca acgtgaatca caagcccagc aacaccaagg tggacaagaa agttgagccc     300
aaatcttgtg acaaaactca cacatgccca ccgtgcccag cacctgaact cctgggggga     360
ccgtcagtct tcctcttccc cccaaaaccc aaggacaccc tcatgatctc ccggacccct     420
gaggtcacat gcgtggtggt ggacgtgagc cacgaagacc ctgaggtcaa gttcaactgg     480
tacgtggacg gcgtggaggt gcataatgcc aagacaaagc cgcgggagga gcagtacaac     540
agcacgtacc gtgtggtcag cgtcctcacc gtcctgcacc aggactggct gaatggcaag     600
gagtacaagt gcaaggtctc caacaaagcc ctcccagccc ccatcgagaa aaccatctcc     660
aaagccaaag ggcagccccg agaaccacag gtgtacaccc tgcccccatc ccgggatgag     720
ctgaccaaga accaggtcag cctgacctgc ctggtcaaag gcttctatcc cagcgacatc     780
gccgtggagt gggagagcaa tgggcagccg gagaacaact acaagaccac gcctcccgtg     840
ctggactccg acggctcctt cttcctctac agcaagctca ccgtggacaa gagcaggtgg     900
cagcagggga acgtcttctc atgctccgtg atgcatgagg ctctgcacaa ccactacaca     960
cagaagagcc tctccctgtc tccgggtaaa                                      990

<210>  19
<211>  106
<212>  PRT
<213>  Homo sapiens

<400>  19
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu Cys
            100                 105

<210>  20
<211>  318
<212>  DNA
<213>  Homo sapiens

<400>  20
actgtggctg caccatctgt cttcatcttc ccgccatctg atgagcagtt gaaatctgga      60
actgcctctg ttgtgtgcct gctgaataac ttctatccca gagaggccaa agtacagtgg     120
aaggtggata acgccctcca atcgggtaac tcccaggaga gtgtcacaga gcaggacagc     180
aaggacagca cctacagcct cagcagcacc ctgacgctga gcaaagcaga ctacgagaaa     240
cacaaagtct acgcctgcga agtcacccat cagggcctga gttcgcccgt cacaaagagc     300
ttcaacaggg gagagtgt                                                   318

<210>  21
<211>  106
<212>  PRT
<213>  Homo sapiens

<400>  21

<210>  22

<211>  318
<212>  DNA
<213>  Homo sapiens

<400>  22
cgtcagccca aggctgcccc ctcggtcact ctgttcccac cctcgagtga ggagcttcaa      60
gccaacaagg ccacactggt gtgtctcata agtgacttct acccgggagc cgtgacagtg     120
gcctggaagg cagatagcag ccccgtcaag gcgggagtgg agaccaccac accctccaaa     180
caaagcaaca acaagtacgc ggccagcagc tacctgagcc tgacgcctga gcagtggaag     240
tcccacaaaa gctacagctg ccaggtcacg catgaaggga gcaccgtgga gaagacagtg     300
gcccctacag aatgttca                                                   318